(19) Europäisches Patentamt
European Patent Office
Office européen des brevets

(11) Publication number: **0 433 909 A2**

# (12) EUROPEAN PATENT APPLICATION

(21) Application number: **90124227.1**

(22) Date of filing: **14.12.90**

(51) Int. Cl.5: **A01N 47/34**, A01N 25/02, A61K 31/17, A61K 31/44, A61K 9/08

(30) Priority: **18.12.89 AU 7893/89**
**01.11.90 AU 65713/90**

(43) Date of publication of application:
**26.06.91 Bulletin 91/26**

(84) Designated Contracting States:
**AT BE CH DE DK ES FR GB GR IT LI LU NL SE**

(71) Applicant: **CIBA-GEIGY AG**
**Klybeckstrasse 141**
**CH-4002 Basel(CH)**

(72) Inventor: **Strong, Michael Blundell**
**6 Menin Road**
**Oakville NSW 2765(AU)**
Inventor: **Kearney, Elaine Mary**
**22 Bounty Avenue**
**Castle Hill NSW 2154(AU)**

(74) Representative: **Zumstein, Fritz, Dr. et al**
**Bräuhausstrasse 4**
**W-8000 München 2(DE)**

## (54) Formulation.

(57) A formulation which solubilizes bio-active substituted benzoylphenyl ureas in water and enhances absorption of and maintains adequate blood levels of said ureas when administered to warm-blooded animals.

EP 0 433 909 A2

## FORMULATION

This invention relates to a formulation which solubilizes bio-active substituted benzoylphenyl ureas in water and enhances absorption of and maintains adequate blood levels of said ureas when administered to warm-blooded animals.

Bio-active substituted benzoylphenyl ureas are a generally known class of compounds. Representative examples of such compounds, their preparation and biological activity are described in the following publications: K. Wellinga et al., J. Agr. Food Chem., Vol. 21, No. 3 (1973), p. 348 to 354; PCT application WO 86/03941, EP application 79,311 and EP application 179,022.

In order to achieve a systemic action of a bio-active substance, liquid formulations provided for introducing said substance into blood vessels or tissue fluids of a living warm-blooded animal should guarantee equal dispersion of the active ingredient in the body of the animal within an appropriate period of time.

In general, bio-active substances which are readily soluble in water are not particularly difficult to formulate. However, bio-active substances not easily dissoluble in water must be incorporated into special liquid systems suitable for solubilization and dispersion of said substances in the micro environment into which the formulation is applied in order to achieve satisfactory bio-availability of the active ingredients.

As is known from the art, bio-active substituted benzoylphenyl ureas are merely sparingly soluble in water. Thus, solubilities of 1-(4-chlorophenyl)-3-(2,6-dichlorobenzoyl)-urea and of 1-(2,6-dichlorobenzoyl)-3-(3,4-dichlorophenyl)-urea do not exceed 1 ppm (K. Wellinga et al., J. Agr. Food Chem. Vol 21, No.3 (1973), p.350, right column, penultimate paragraph), and solubility in water of 1-(4-chlorophenyl)-3-(2,6-difluorobenzoyl)urea is about 0.3 ppm ("The Merck Index", 9th Ed. (1976), Merck & Co., Rahway, N.J., U.S.A., p. 416, "Difluron").

Thus, in order to achieve solubilization and uniform dispersion of the bio-active substituted benzoylphenyl ureas in the animal to be treated in an appropriate period of time and to attain adequate tolerability by the tissue adjacent to the zone of treatment, a special liquid system is required which is allowed to merely contain physiologically acceptable constituents.

The instant invention relates to a process for extending the solubilization in water of bio-active substituted benzoylphenyl ureas which are sparingly soluble in water, to a method for achieving enhanced absorption of said ureas in warm-blooded animals and to a formulation containing at least one bio-active substituted benzoylphenyl urea which is sparingly soluble in water.

According to a first embodiment of the present invention there is provided a formulation comprising:

(i) at least one bio-active substituted benzoylphenyl urea which is sparingly soluble in water;
(ii) at least one N-substituted pyrrolidinone solvent;
(iii) at least one ester of polyethylene glycol; and
(iv) at least one non-ionic surfactant selected from an ethoxylated ester of a fatty acid or a triglyceride.

According to a second embodiment of the present invention there is provided a method of solubilizing in water a bio-active substituted benzoylphenyl urea which is sparingly soluble in water or a mixture of such ureas by combining said urea or said mixture of ureas with

(ii) at least one N-substituted pyrrolidinone solvent;
(iii) at least one ester of polyethylene glycol; and
(iv) at least one non-ionic surfactant selected from an ethoxylated ester of a fatty acid or a triglyceride.

According to a third embodiment of the present invention there is provided a method for enhancing absorption and maintaining adequate blood levels in warm-blooded animals of bio-active substituted benzoylphenyl ureas which are sparingly soluble in water, comprising administering to said warm-blooded animals either orally, directly into the gastro-intestinal tract or parenterally a formulation comprising:

(i) at least one bio-active substituted benzoylphenyl urea which is sparingly soluble in water;
(ii) at least one N-substituted pyrrolidinone solvent;
(iii) at least one ester of polyethylene glycol; and
(iv) at least one non-ionic surfactant selected from an ethoxylated ester of a fatty acid or a triglyceride.

Suitable bio-active substituted benzoylphenyl ureas which are sparingly soluble in water and which may be used in the present invention are preferably those of formula I

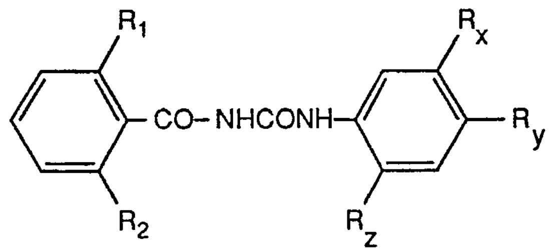 (I)

wherein

$R_1$ is hydrogen, methyl, fluorine, chlorine, bromine, methoxy or methylthio,

$R_2$ is hydrogen, methyl, fluorine, chlorine, bromine, methoxy or methylthio, and

i) $R_x$ is chlorine, $R_y$ is -O-$R_3$, wherein $R_3$ is halo-$C_1$-$C_7$alkyl containing 1 to 15 halogen atoms or $R_3$ is halo-$C_3$-$C_7$cycloalkyl containing 1 to 13 halogen atoms, and $R_z$ is chlorine, or

ii)

$R_x$ is 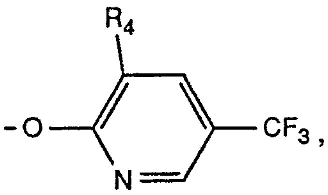

wherein $R_4$ is hydrogen or chlorine, $R_y$ is hydrogen, methyl, fluorine, chlorine or bromine, and $R_z$ is hydrogen, or

iii) $R_x$ is hydrogen $R_y$ is chlorine $R_z$ is hydrogen.

Particularly preferred are compounds of formula I, group i), on account of their action as ectoparasiticidal active substances, wherein

a) $R_1$ is hydrogen, methyl, fluorine, chlorine, methoxy or methylthio, $R_2$ is methyl, fluorine, chlorine, methoxy or methylthio, and $R_3$ is halo-$C_1$-$C_7$alkyl containing 1 to 15 halogen atoms, or $R_3$ is halo-$C_3$-$C_7$cycloalkyl containing 1 to 13 halogen atoms;

b) $R_1$ is hydrogen, methyl, fluorine, chlorine, methoxy or methylthio, $R_2$ is methyl, fluorine, chlorine, methoxy or methylthio, and $R_3$ is halo-$C_5$-$C_7$alkyl containing 1 to 15 halogen atoms;

c) $R_1$ is hydrogen, methyl, fluorine, chlorine, methoxy or methylthio, $R_2$ is methyl, fluorine, chlorine, methoxy or methylthio, and $R_3$ is halo-$C_1$-$C_3$alkyl containing 1 to 7 halogen atoms;

d) i) $R_1$ is hydrogen and $R_2$ is methyl, methoxy or methylthio, or ii) $R_1$ is fluorine and $R_2$ is methyl, fluorine, methoxy or methylthio, or iii) $R_1$ is chlorine and $R_2$ is methyl, chlorine, methoxy or methylthio, and $R_3$ is halo-$C_1$-$C_7$alkyl containing 1 to 15 halogen atoms, or $R_3$ is halo-$C_3$-$C_7$cycloalkyl containing 1 to 13 halogen atoms;

e) i) $R_1$ and $R_2$ are simultaneously fluorine, or ii) $R_1$ and $R_2$ are simultaneously chlorine, or iii) $R_1$ and $R_2$, independently of each other, are methyl, methoxy or methylthio, and $R_3$ is halo-$C_1$-$C_7$alkyl containing 1 to 15 halogen atoms, or $R_3$ is halo-$C_3$-$C_7$cycloalkyl containing 1 to 13 halogen atoms;

f) $R_1$ is hydrogen, methyl, fluorine, chlorine, methoxy or methylthio, $R_2$ is methyl, fluorine, chlorine, methoxy or methylthio, and $R_3$ is $-CHF_2$, $-CF_3$, $-CF_2CHF_2$, $-CH_2CF_3$, $-CF_2CHFCl$, $-CF_2CHCl_2$, $-CF_2CCl_3$, $-CF_2CHBr_2$, $-CF_2CHFBr$, $-CH_2CHBrCH_2Br$, $-CF(CF_3)CHFCF_3$, $-CF_2CHFCF(CF_3)_2$, $-CF_2CHF(CF_2)_4CF_3$, $-CF_2CHFCF_3$, or

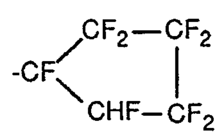 ,

in particular those compounds wherein $R_3$ is one of the radicals $-CF_2CHF_2$, $-CF_2CHCl_2$, $-CF_2CHFCl$, $-CF_2CCl_3$ or $-CF_2CHFCF_3$, especially the radical $-CF_2CHFCF_3$;

g) $R_1$ is hydrogen, fluorine, chlorine or methoxy, $R_2$ is fluorine, chlorine or methoxy, and $R_3$ is

$-CF_2CHFCF_3$;

h) $R_1$ is hydrogen, fluorine or chlorine, $R_2$ is fluorine, chlorine or methoxy, and $R_3$ is $-CF_2CHFCF_3$;

i) $R_1$ and $R_2$ are simultaneously fluorine, chlorine or methoxy, and $R_3$ is $-CF_2CHFCF_3$;

k) $R_1$ is hydrogen, fluorine or chlorine, $R_2$ is fluorine or chlorine, and $R_3$ is $-CF_2CHFCF_3$;

l) $R_1$ is hydrogen or fluorine, $R_2$ is fluorine or chlorine, and $R_3$ is $-CF_2CHFCF_3$; or

m) $R_1$ is fluorine, $R_2$ is fluorine, and $R_3$ is $-CF_2CHFCF_3$.

Particularly preferred are compounds of formula I, group ii), on account of their action as ectoparasitical active substances; wherein

a) $R_1$, $R_2$ and $R_y$, independently of each other, are hydrogen, methyl, fluorine, chlorine or bromine, and $R_4$ is hydrogen or chlorine;

b) $R_1$ and $R_2$, independently of each other, are hydrogen, methyl, fluorine, chlorine or bromine, $R_y$ is methyl, fluorine, chlorine or bromine, and $R_4$ is chlorine;

c) $R_1$ is methyl, fluorine or chlorine, $R_2$ is hydrogen, methyl, fluorine or chlorine, $R_y$ is hydrogen, methyl, fluorine, chlorine or bromine, and $R_4$ is hydrogen or chlorine;

d) $R_1$ is fluorine or chlorine, $R_2$ is hydrogen or fluorine, $R_y$ is hydrogen, methyl or chlorine, and $R_4$ is hydrogen or chlorine;

e) $R_1$ is fluorine, $R_2$ is fluorine, $R_y$ is hydrogen, methyl, fluorine, chlorine or bromine, and $R_4$ is hydrogen or chlorine;

f) $R_1$ is fluorine, $R_2$ is fluorine, $R_y$ is chlorine, and $R_4$ is chlorine.

Within the scope of this invention, the most preferred compounds are N-(4-chlorophenyl-N'-(2,6-difluorobenzoyl)urea, N-(2,5-dichloro-4-(1,1,2,3,3,3-hexafluoropropoxy)phenyl)-N'-(2,6-difluorobenzoyl)urea and N-(4-chloro-3-(3-chloro-5-trifluoromethyl-pyridyl-2-oxy)phenyl)-N'-(2,6-difluorobenzoyl) urea, particularly N-(2,5-dichloro-4-(1,1,2,3,3,3-hexafluoropropoxy)phenyl)-N'-(2,6-difluorobenzoyl)urea and, most particularly, N-(4-chloro-3-(3-chloro-5-trifluoromethyl-pyridyl-2-oxy)phenyl)-N'-(2,6-difluorobenzoyl)urea.

Suitable N-substitued pyrrolidinone solvents are physiologically acceptable compounds, such as, for example, the alkyl pyrrolidinones, such as N-methyl-2-pyrrolidinone (NMP), N-ethyl-2-pyrrolidinone, N-isopropyl-2-pyrrolidinone and N-octyl-2-pyrrolidinone, N-cyclohexyl-2-pyrrolidinone and N-(2-hydroxyethyl)-2-pyrrolidinone, particularly N-methyl-2-pyrrolidinone.

Many polyethylene glycol esters are capable of being used in the present invention. Generally, the polyethylene glycol in said esters has a molecular weight of 100 to 6000, preferably 100 to 400. Suitable polyethylene glycol esters are particularly those of formulae II, III and IV:
compounds of formula II are

$$CH_3\text{-}(CH_2)_a\text{-}CO\text{-}(OCH_2CH_2)_n\text{-}O\text{-}R_6 \qquad \text{(II)}$$

wherein i) $R_6$ is hydrogen, a is 8 to 16 and n is 4 to 20, or ii) $R_6$ is $-CO-(CH_2)_b-CH_3$, b is 8 to 16, a is 8 to 16 and n is 4 to 150; these compounds are derived in particular from fatty acids such as lauric acid and stearic acid;
compounds of formula III are

$$CH_3\text{-}(CH_2)_c\text{-}CH{=}CH\text{-}(CH_2)_d\text{-}CO\text{-}(OCH_2CH_2)_m\text{-}OH \qquad \text{(III)}$$

wherein m is 4 to 20, c is 6 to 8 and d is 6 to 8; preferably, c and d each are 7;
compounds of formula IV are

$$HO\text{-}(CH_2CH_2O)_e\text{-}CO\text{-}(CH_2)_k\text{-}CO\text{-}(OCH_2CH_2)_f\text{-}OH \qquad \text{(IV)}$$

wherein k is 3 to 6; preferably 4, e is 4 to 20 and f is 4 to 20.

Further polyethylene glycol esters of cycloaliphatic carboxylic acids related to abietic acid are also suitable.

Preferred polyethylene glycol esters are those with fatty acids. Such compounds are commercially available, for example, under the product names Atlas ®, Kessco, Lipopeg, Pegosperse® and Witconol. Particularly suitable with respect to the instant invention are the following polyethylene glycol esters (most

of them published, for example, in "Chemical Product Desk Reference", M. and I. Ash, Chemical Publishing Co., Inc., New York, NY, 1990; Kirk-Othmer, "Encyclopedia of Chemical Technicology", 3rd Ed., Vol. 22, 1983, John Wiley & Sons, New York, Chichester, Brisbane, Toronto, Singapore, p.369):

| | |
|---|---|
| Kessco PEG 200 Monolaurate (PEG-4 laurate) | HLB 9.8* |
| Kessco PEG 200 Dilaurate (PEG-4 dilaurate) | HLB 5.9* |
| Kessco PEG 400 Dilaurate (PEG-8 dilaurate) | HLB 9.8* |
| Kessco PEG 400 Monolaurate (PEG-8 laurate) | HLB 13.1* |
| Kessco PEG 400 Dioleate (PEG-8 dioleate) | HLB 8.5* |
| Kessco PEG 400 Monooleate (PEG-8 oleate) | HLB 11.4* |
| Kessco PEG 400 Distearate (PEG-8 distearate) | HLB 8.0* |
| Kessco PEG 400 Monostearate (PEG-8 stearate) | HLB 11.6* |
| Kessco PEG 600 Monolaurate (PEG-12 laurate) | HLB 14.9* |

* Kessco esters (Stepan; Armak Co., Industrial Chemical Division)

| | |
|---|---|
| Pegosperse® 400 MS (PEG-8 stearate) | HLB 11.2 ± 0.5** |
| Pegosperse® 400 MO (PEG-8 oleate) | HLB 11.0 ± 0.5** |
| Pegosperse® 400 ML (PEG-8 laurate) | HLB 13.9 ± 0.5** |
| Pegosperse® 600 ML (PEG-12 laurate) | HLB 14.6 ± 0.5** |

** (Glyco Chemicals Inc.; Lonza)

Witconol H-31A (PEG-8 oleate)***
Witconol L32-45 (PEG-150 distearate)***

*** (Witco Chemical Corp.)

From the polyethylene glycol esters, PEG 200 dilaurate (PEG-4 dilaurate) is particularly preferred.

The non-ionic surfactant carries no discrete charge when dissolved in aqueous media and is selected from ethylene glycol and diethylene glycol mono and diesters of fatty acids, in particular of the formulae V, VI and VII: compounds of formula V are

$$CH_3\text{-}(CH_2)_g\text{-}CO\text{-}(OCH_2CH_2)_p\text{-}OH \qquad (V)$$

wherein g is 8 to 16 and p is 1 or 2;
compounds of formula VI are

$$CH_3\text{-}(CH_2)_q\text{-}CH{=}CH\text{-}(CH_2)_r\text{-}CO\text{-}(OCH_2CH_2)_s\text{-}OH \qquad (VI)$$

wherein q is 6 to 8; r is 6 to 8, and s is 1 or 2.
compounds of formula VII are

$$CH_3\text{-}(CH_2)_h\text{-}CO\text{-}OCH_2CH_2O\text{-}CO\text{-}(CH_2)_i\text{-}CH_3 \qquad (VII)$$

wherein h and i each are 8 to 16,
or from triglycerides. Suitable mixtures of triglycerides are represented by castor oil, coconut oil or are constituents of tallow. In some instances, it is of value to hydrogenate some or all of the unsaturated bonds in the aliphatic chains derived from the acid part of the molecule. Particularly suitable non-ionic surfactants are compounds selected from the group consisting of ethoxylated triglyceride, such as ethoxylated castor oil. Especially preferred non-ionic surfactants are:

| | |
|---|---|
| Cremophor® EL (PEG-36 castor oil) | HLB 12-14 |
| Cremophor® RH 40 (PEG-40 hydrogenated castor oil) | HLB 14-16 |
| Atlas® G-1284 (POE triglyceride) | HLB 13.1 |

(Cremophor®:BASF, described, for example, in "Chemical Products Desk Reference", M. and I. Ash, Chemical Publishing Co., Inc., New York, NY, 1990, p.166; Atlas®: Atlas Refinery; ICI Speciality Chemicals, described, for example, in "Chemical Products Desk Reference", M. and I. Ash, Chemical Publishing Co., Inc., New York, NY, 1990, p. 78; "1990 McCutcheon's Emulsifiers & Detergents International Edition", The Manufacturing Confectioner Publishing Co., 175 Rock Road, Glen Rock, NJ 07452, USA).

Particularly suitable formulations of the instant invention are those which, besides at least one bio-active substituted benzoylphenyl urea which is sparingly soluble in water, at least one N-substituted pyrrolidinone solvent, at least one ester of polyethylene glycol and at least one non-ionic surfactant selected from an ethoxylated ester of a fatty acid or a triglyceride, contain crystal modifiers such as carboxymethyl cellulose or ethyl cellulose and/or epoxy-based stabilizers such as epoxidised soybean oil (suitable, for example, is epoxidised soybean oil, EO 6.0 - 6.3 %, such as Reoplast® 39 (CIBA-GEIGY AG), described on technical instruction sheet of CIBA-GEIGY AG).

Solubilization is achieved by the synergistic action of the pyrrolidinone solvent, the ester of polyethylene glycol and the non-ionic surfactant, especially ethoxylated triglycerides and particularly ethoxylated castor oil. This provides:

a) a phase in which the bio-active molecule is solubilized as a concentrate;
b) controlled release of the active substance when the product comes in contact with water;
c) energy release, when water and the formulation are in contact, sufficient for spontaneous dispersion to occur;
d) a solution viscosity sufficient to influence the rate of dispersion of the mixture in water;
e) prolonged inhibition of the precipitation or crystallization of the bio-active substance from the water dispersed product.

In some cases, the presence of ethyl cellulose turns out to be advantageous since ethyl cellulose acts as an inhibitor of precipitation of the bio-active substance in the concentrate itself and in dilution of the concentrate with water.

In general, the formulations according to the instant invention contain the bio-active substituted benzoylphenyl urea in a concentration of 0.1 to 25 % by weight, in particular 0.5 to 20 % by weight.

Preferred concentrations of the N-substituted pyrrolidinone solvent are 5 to 25 % by weight, in particular 8 to 15 % by weight.

Generally, the concentration of polyethylene glycol ester amounts to 30 to 60 % by weight, particularly to 35 to 50 % by weight.

Preferred concentrations of a non-ionic surfactant selected from an ethoxylated ester of a fatty acid or a triglyceride are 20 to 60 % by weight, particularly 20 to 45 % by weight.

Particularly suitable are formulations containing 0.1 to 25 % by weight of at least one bio-active substituted benzoylphenyl urea which is sparingly soluble in water, 5 to 25 % by weight of at least one N-substituted pyrrolidinone solvent, 30 to 60 % by weight of at least one ester of polyethylene glycol and 20 to 60 % by weight of at least one non-ionic surfactant selected from an ethoxylated ester of a fatty acid or a triglyceride. In addition to the above mentioned ingredients, some preferred formulations also contain from

0.1 to 0.3 % by weight of ethyl cellulose.

Preferably, the formulation contains 0.1 to 25 % by weight of at least one bio-active substituted benzoylphenyl urea which is sparingly soluble in water, particularly those of formula I and especially N-(4-chlorophenyl-N'-(2,6-difluorobenzoyl)urea, N-(2,5-dichloro-4-(1,1,2,3,3,3-hexafluoropropoxy)phenyl)-N'-(2,6-difluorobenzoyl)urea or N-(4-chloro-3-(3-chloro-5-trifluoromethyl-pyridyl-2-oxy)phenyl)-N'-(2,6-difluorobenzoyl) urea and most preferred N-(2,5-dichloro-4-(1,1,2,3,3,3-hexafluoropropoxy)phenyl)-N'-(2,6-difluorobenzoyl)urea and N-(4-chloro-3-(3-chloro-5-trifluoromethyl-pyridyl-2-oxy)phenyl)-N'-(2,6-difluorobenzoyl)urea, 5 to 25 % by weight of at least one substitued pyrrolidinone solvent, particularly N-methyl-2-pyrrolidinone, 30 to 60 % by weight of at least one ester of polyethylene glycol,particularly fatty acid esters of polyethylene glycol containing 4 to 150 EO, and 20 to 60 % by weight of at least one non-ionic surfactant selected from an ethoxylated ester of a fatty acid or a triglyceride, particularly ethoxylated castor oil, and 0 to 0.3 % by weight of ethyl cellulose such as, for example, ethyl cellulose N-300 (Hercules Inc.; N-300: 48-49.5 % ethoxyl content; nominal viscosity in centipoises (cps) for a 5 % concentration by weight in 80:20 toluene:ethanol by weight, determined on a sample dried 30 min at 100 °C, is in the range of from 250 to 350 cps; "Chemical and Physical Properties of Hercules' Ethyl Cellulose", 1989, pages 1 and 2).

The formulation may be applied by parenteral injection, I/R injection (intrarumenal injection), oral dosing or topically.

The formulations according to the instant invention are suitable for combatting ectoparasites in warm-blooded animals, especially mammals and preferably domestic animals and productive livestock. Animals to be treated are, for example, sheep, pigs, goats, cattle, horses, donkeys, buffalos, camels, reindeers, caribous, dogs, cats and guinea pigs.

Ectoparasites to be combatted include, for example, fleas, lice, mites, ticks and flies including parasitizing fly larvae as, for example, the causative organisms of myiasis.

Particularly to be mentioned are ectoparasites of the order Acarina, especially members of the families Ixodidae, Dermanyssidae, Sarcoptidae, Psoroptidae and Trombiculidae, of the order Mallophaga and Siphonaptera, of the family Haemotopinidae belonging to the order Anoplura, and of the order Diptera, especially members of the families Muscidae, Glossinidae, Calliphoridae, Oestridae, Tabanidae, Hippoboscidae and Gasterophilidae.

The invention also relates to a method for prophylactically protecting and/or for curative treatment of warm-blooded animals against ectoparasites. The inventive formulations may be administered as a single dose or repeatedly, the single administration being performed in general with 1 to 20 mg, preferably 1 to 10 mg, of the bio-active substituted benzoylphenyl urea per kg of body weight depending on the parasite to be controlled.

The invention is further illustrated by the following examples.

With respect to some constituents of formulations used in the following examples, further information about particularly suitable commercial products is given below, without, however, limiting the examples to said products.

For said constituents, the following information is given (as far as not already mentioned otherwise): name of the product/commercial distributor/reference/present example and/or composition wherein the constituent is named:

| | |
|---|---|
| POE triglyceride - Atlas G 1284 | P-1, P-2 |
| ethyl cellulose - Ethyl Cellulose N-300/Hercules Inc. | P-1, P-4 |
| N-octylpyrrolidone - Surfadone LP-100/GAF/<br>Tensid-Taschenbuch, H. Stache and K. Kosswig,<br>Carl Hanser Verlag München, Wien, 1990, p. 336 | P-2 |
| PEG-36 castor oil - Cremophor® EL | P-3, P-4 |

| | |
|---|---|
| PEG-2 methyl ether - Dowanol® DMP/DOW/Chemical Products Desk Reference, M. and I. Ash, Chemical Publishing Co., Inc., New York, NY, 1990, p. 215 | B-1, A |
| PEG-40 hydrogenated castor oil - Cremophor® RH 40 | B-1, B |
| microcrystalline cellulose - Avicel P-102/FMC/Chemical Products Desk Reference, M. and I. Ash, Chemical Publishing Co., Inc., New York, NY, 1990, p. 83; Römpp Chemie Lexikon, 9th Ed., Georg Thieme Verlag, Stuttgart, New York, 1989, p. 319 | B-2, D |
| pregelatinized starch - Starch 1500/Concise Chemical and Technological Dictionary, H. Bennett, Chemical Publishing Co., Inc., New York, NY, 1986, p. 1065 | B-2, D |
| polysorbate 20 - Tween® 20/ICI Am./Chemical Products Desk Reference, M. and I. Ash, Chemical Publishing Co., Inc., New York, NY, 1990, p. 885; Römpps Chemie-Lexikon, 8th Ed., 1988, p. 4394 | B-2, D |
| carboxymethylcellulose sodium that has been internally cross-linked - Ac-Di-Sol/FMC/Gardner's Chemical Synonyms and Trade Names, 9th Ed., 1987, J. Pearce, Gower Technical Press, p. 3 | B-2, D |

Example P-1: Preparation of formulation C-1 according to the instant invention

| | |
|---|---|
| N-(4-chloro-3-(3-chloro-5-trifluoromethyl-pyridinyl-2-oxy)-phenyl)-N'-(2,6-difluorobenzoyl)urea | 2.50 % w/w |
| PEG-200 dilaurate | 45.00 % w/w |
| POE triglyceride | 42.50 % w/w |
| ethyl cellulose N-300 | 0.15 % w/w |
| N-methyl-2-pyrrolidinone | 9.85 % w/w |

Ethyl cellulose is added to N-methyl-2-pyrrolidinone at 80 °C and the mixture is stirred until a solution is obtained and then cooled to room temperature. N-(4-chloro-3-(3-chloro-5-trifluoromethyl-pyridinyl-2-oxy)-phenyl)-N'-(2,6-difluorobenzoyl)urea is added to said solution and the mixture is stirred until the added material has dissolved. To the solution thus formed, PEG-200 dilaurate is added and the mixture is stirred until homogeneous. Finally, POE triglyceride is added and the mixture is stirred until homogeneous.

Example P-2: Preparation of formulation C-2 according to the instant invention

| | |
|---|---|
| N-(4-chloro-3-(3-chloro-5-trifluoromethyl-pyridinyl-2-oxy)-phenyl)-N'-(2,6-difluorobenzoyl)urea | 2.50 % w/w |
| PEG-200 dilaurate | 40.00 % w/w |
| POE triglyceride | 37.50 % w/w |
| N-octylpyrrolidone | 10.00 % w/w |
| N-methyl-2-pyrrolidinone | 10.00 % w/w |

N-(4-chloro-3(3-chloro-5-trifluoromethyl-pyridinyl-2-oxy)phenyl)-N'-(2,6-d ifluorobenzoyl)urea is dissolved in N-methyl-2-pyrrolidinone and the mixture is stirred until a solution is obtained. To said solution, N-octylpyrrolidone is added followed by addition of PEG-200 dilaurate. The mixture is stirred until homogeneous. Then, POE triglyceride is added and the mixture is stirred until homogeneous.

Example P-3: Preparation of formulation C-3 according to the instant invention

| | |
|---|---|
| N-(2,5-dichloro-4-(1,1,2,3,3,3-hexafluoropropoxy)phenyl)-N'-(2,6-difluorobenzoyl)urea | 20.00 % w/w |
| PEG-200 dilaurate | 45.00 % w/w |
| PEG-36 castor oil | 22.50 % w/w |
| N-methyl-2-pyrrolidinone | 12.50 % w/w |

N-(2,5-dichloro-4-(1,1,2,3,3,3-hexafluoropropoxy)phenyl)-N'-(2,6-difluorobenzoyl)urea is dissolved in N-methyl-2-pyrrolidinone. To the solution thus obtained PEG-200 dilaurate is added and the mixture is stirred until homogeneous. Then, PEG-36 castor oil is added and the mixture is stirred until homogeneous.

Example P-4: Preparation of formulation C-4 according to the instant invention

| | |
|---|---|
| N-(4-chloro-3-(3-chloro-5-trifluoromethyl-pyridinyl-2-oxy)-phenyl)-N'-(2,6-difluorobenzoyl)urea | 2.50 % w/w |
| PEG-200 dilaurate | 45.00 % w/w |
| PEG-36 castor oil | 42.50 % w/w |
| ethyl cellulose N-300 | 0.15 % w/w |
| N-methyl-2-pyrrolidinone | 9.85 % w/w |

Ethyl cellulose is added to N-methyl-2-pyrrolidinone at 80 °C and the mixture is stirred until a solution has been formed, which is cooled to room temperature. To said solution N-(4-chloro-3-(3-chloro-5-trifluoromethyl-pyridinyl-2-oxy)phenyl)-N'-(2,6-difluorobenzoyl)urea is added and the mixture is stirred until the added material has dissolved. Then PEG-200 dilaurate is added and the mixture is stirred until homogeneous. PEG-36 castor oil is added to the homogeneous material and the mixture is stirred until homogeneous.

Example B-1: Comparative test

The following formulations are injected subcutaneously into cattle at a concentration of 1 mg per kg of body weight:

| | |
|---|---|
| Comparative formulation A: | |
| N-(4-chloro-3-(3-chloro-5-trifluoromethyl-pyridinyl-2-oxy)-phenyl)-N'-(2,6-difluorobenzoyl)urea | 2.5 g |
| PPG-2 methyl ether qs. ad. | 100 ml |

N-(4-chloro-3-(3-chloro-5-trifluoromethyl-pyridinyl-2-oxy)phenyl)-N'-(2,6-difluorobenzoyl)urea is dissolved in PEG-2 methyl ether under stirring and, if the volume obtained does not amount to 100 ml, PEG-2 methyl ether is added until 100 ml are reached.

| | |
|---|---|
| Comparative formulation B: | |
| N-(4-chloro-3-(3-chloro-5-trifluoromethyl-pyridinyl-2-oxy)-phenyl)-N'-(2,6-difluorobenzoyl)urea | 2.5 g |
| PEG-40 hydrogenated castor oil | 40.0 g |
| N-methyl-2-pyrrolidinone qs. ad. | 100 ml |

N-(4-chloro-3-(3-chloro-5-trifluoromethyl-pyridinyl-2-oxy)phenyl)-N'-(2,6-difluorobenzoyl)urea is dissolved in N-methyl-2-pyrrodidinone and stirred. Then, PEG-40 hydrogenated castor oil is added and the mixture is stirred. If the volume obtained does not amount to 100 ml, N-methyl-2-pyrrolidinone is added until 100 ml are reached.

Formulation C-4 of example P-4.

The concentration of N(4-chloro-3-(3-chloro-5-trifluoromethyl-pyridinyl-2-oxy)phenyl)-N'-(2,6-difluorobenzoyl)urea in the cattle's blood is determined at 1, 3, 7, 10, 14, 21, 28, 35, 42 and 56 days after treatment. The results are shown in Table 1 (ng of active substance per ml of plasma; ng defines 1 x $10^{-9}$g):

TABLE 1

| Formulation | d.a.t. / N | 1 | 3 | 7 | 10 | 14 | 21 | 28 | 35 | 42 | 56 |
|---|---|---|---|---|---|---|---|---|---|---|---|
| | | concentration of active compound in plasma (ng/ml) | | | | | | | | | |
| Comparative Formulation A | 746 | 0 | 1 | 3 | 4 | 8 | 9 | 9 | 10 | 11 | 7 |
| | 748 | 0 | 2 | 2 | 3 | 5 | 3 | 8 | 7 | 3 | 5 |
| | 753 | 0 | 2 | 3 | 5 | 6 | 9 | 8 | 6 | 7 | 5 |
| | 756 | 4 | 6 | 12 | 21 | 27 | 26 | 27 | 16 | 12 | 7 |
| | Mean | 1 | 3 | 5 | 8 | 12 | 12 | 13 | 10 | 8 | 6 |
| | SD | 2 | 2 | 5 | 9 | 10 | 10 | 9 | 5 | 4 | 2 |
| Comparative Formulation B | 738 | 23 | 15 | 14 | 19 | 26 | 27 | 20 | 21 | 14 | 12 |
| | 745 | 25 | 20 | 17 | 22 | 23 | 21 | 19 | 16 | 12 | 8 |
| | 749 | 25 | 16 | 21 | 28 | 25 | 26 | 19 | 17 | 12 | 11 |
| | 750 | 16 | 7 | 8 | 12 | 16 | 18 | 15 | 12 | 13 | 10 |
| | Mean | 22 | 15 | 15 | 20 | 23 | 23 | 18 | 17 | 13 | 10 |
| | SD | 4 | 6 | 5 | 7 | 5 | 4 | 2 | 4 | 1 | 2 |
| Formulation C-4 | 737 | 74 | 33 | 27 | 27 | 26 | 17 | 15 | 15 | 10 | 10 |
| | 744 | 98 | 43 | 33 | 30 | 36 | 22 | 19 | 16 | 12 | 8 |
| | 755 | 102 | 34 | 25 | 26 | 23 | 21 | 16 | 14 | 12 | 9 |
| | 768 | 66 | 27 | 16 | 18 | 17 | 15 | 17 | 12 | 10 | 8 |
| | Mean | 85 | 34 | 25 | 25 | 26 | 19 | 17 | 14 | 11 | 9 |
| | SD | 18 | 7 | 7 | 5 | 8 | 3 | 2 | 2 | 1 | 1 |

Note: 0 = below detectable limit of 1 ng  Recovery at 10 ng = 95 ± 8 % (n=24)
SD = statistical deviation

N. = Number of the test animal
d.a.t. = day after treatment

As can be seen from the results given in Table 1, the cattle treated with formulation C-4 of the present invention shows a much better absorption of N-(4-chloro-3-(3-chloro-5-trifluoromethyl-pyridinyl-2-oxy)-phenyl)-N'-(2,6-difluorobenzoyl)urea than those treated with the comparative compositions A and B.

Example 2-B: Comparative test

A comparison is made between the following tablet formulation (composition D) and a solution of the

instant invention.

| | |
|---|---|
| Composition D: | |
| N-(2,5-dichloro-4-(1,1,2,3,3,3-hexafluoropropoxy)phenyl)-N'-(2,6-difluorobenzoyl)urea | 50.00 % w/w |
| lactose | 20.25 % w/w |
| microcrystalline cellulose | 20.25 % w/w |
| pregelatinized starch | 5.00 % w/w |
| polysorbate 20 | 2.00 % w/w |
| carboxymethylcellulose sodium that has been internally cross-linked | 2.00 % w/w |
| magnesium stearate | 0.50 % w/w |

The constituents given above are thoroughly mixed together and the mixture is formed to tablets.

Three consecutive treatments are carried out at six dogs. Treatments 1 and 2 are made with composition D, and treatment 3 is made with the composition C-3 (solution) according to example P-3. Treatment 2 is made four weeks after treatment 1, and treatment 3 is made four weeks after treatment 2. Increases in the concentration of N-(2,5-dichloro-4-(1,1,2,3,3,3-hexafluoropropoxy)phenyl)-N'-(2,6-difluorobenzoyl)urea found in the plasma of the dogs two days after treatment compared with the levels found immediately before treatment are shown in table 2 (ng of active substance per ml of plasma):

TABLE 2:

ng of active substances per ml of plasma

| Dog No. | Treatment 1 (Tablet) | Treatment 2 (Tablet) | Treatment 3 (Liquid) |
|---|---|---|---|
| 6 | 107 | 70 | 206 |
| 7 | 48 | 49 | 399 |
| 12 | 64 | 54 | 279 |
| 32 | 41 | 50 | 433 |
| 33 | 61 | 44 | 281 |
| 34 | 97 | 148 | 258 |
| MEAN | 70 | 69 | 309 |
| ± SD | 27 | 40 | 88 |

As can be seen from the results, the liquid formulation is clearly superior to the tablet formulation.

## Claims

1. A formulation suitable for combatting ectoparasites in warm-blooded animals, which comprises

(i) at least one bio-active substituted benzoylphenyl urea which is sparingly soluble in water;
(ii) at least one N-substituted pyrrolidinone solvent;
(iii) at least one ester of polyethylene glycol; and
(iv) at least one non-ionic surfactant selected from an ethoxylated ester of a fatty acid or a triglyceride.

**2.** The formulation according to claim 1 which contains, as bio-active substituted benzoylphenyl urea which is sparingly soluble in water, a compound of formula I

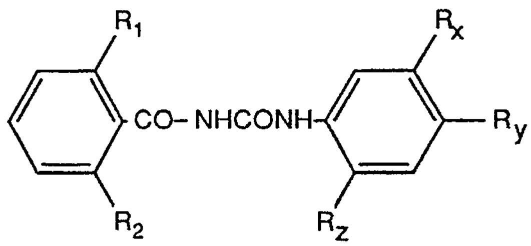

(I)

wherein
$R_1$ is hydrogen, methyl, fluorine, chlorine, bromine, methoxy or methylthio,
$R_2$ is hydrogen, methyl, fluorine, chlorine, bromine, methoxy or methylthio, and
i) $R_x$ is chlorine, $R_y$ is -O-$R_3$, wherein $R_3$ is halo-$C_1$-$C_7$alkyl containing 1 to 15 halogen atoms or $R_3$ is halo-$C_3$-$C_7$cycloalkyl containing 1 to 13 halogen atoms, and $R_z$ is chlorine, or
ii)

$R_x$ is 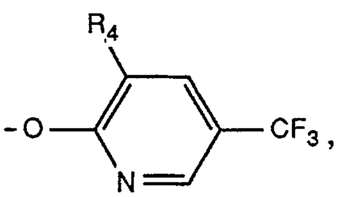

wherein $R_4$ is hydrogen or chlorine, $R_y$ is hydrogen, methyl, fluorine, chlorine or bromine, and $R_z$ is hydrogen, or
iii) $R_x$ is hydrogen $R_y$ is chlorine $R_z$ is hydrogen.

**3.** The formulation according to claim 2 which contains a compound of formula I wherein
$R_1$ is hydrogen, methyl, fluorine, chlorine, methoxy or methylthio,
$R_2$ is methyl, fluorine, chlorine, methoxy or methylthio,
$R_x$ is chlorine,
$R_y$ is -O-$R_3$, wherein $R_3$ is halo-$C_1$-$C_7$alkyl containing 1 to 15 halogen atoms, or $R_3$ is halo-$C_3$-$C_7$cycloalkyl containing 1 to 13 halogen atoms, and
$R_z$ is chlorine.

**4.** The formulation according to claim 2 which contains a compound of formula I, wherein
$R_1$, $R_2$ and $R_y$, independently of each other, are hydrogen, methyl, fluorine, chlorine or bromine,

$R_x$ is 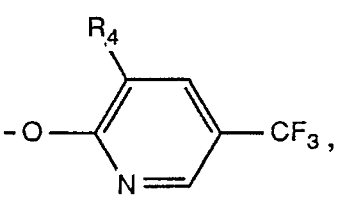

wherein $R_4$ is hydrogen or chlorine, and $R_z$ is hydrogen,

5. The formulation according to claim 2 which contains a compound of formula I, wherein
$R_1$ is hydrogen, methyl, fluorine, chlorine, bromine, methoxy or methylthio,
$R_2$ is hydrogen, methyl, fluorine, chlorine, bromine, methoxy or methylthio,
$R_x$ is hydrogen,
$R_y$ is chlorine and
Rz is hydrogen.

6. The formulation according to claim 2 which contains a compound of formula I selected from the group consisting of N-(4-chlorophenyl-N'-(2,6-difluorobenzoyl)urea, N-(2,5-dichloro-4-(1,1,2,3,3,3-hexafluoropropoxy)phenyl)-N'-(2,6-difluorobenzoyl)urea and N-(4-chloro-3-(3-chloro-5-trifluoromethyl-pyridyl-2-oxy)phenyl)-N'-(2,6-difluorobenzoyl)urea.

7. The formulation according to claim 6 which contains the compound of formula I N-(4-chloro-3-(3-chloro-5-trifluoromethyl-pyridyl-2-oxy)phenyl)-N'-(2,6-difluorobenzoyl)urea.

8. The formulation according to claim 1 which contains an N-substitued pyrrolidinone solvent selected from the group consisting of N-methyl-2-pyrrolidinone, N-ethyl-2-pyrrolidinone, N-isopropyl-2-pyrrolidinone, N-octyl-2-pyrrolidinone, N-cyclohexyl-2-pyrrolidinone and N-(2-hydroxyethyl)-2-pyrrolidinone.

9. The formulation according to claim 1 which contains the N-substituted pyrrolidinone solvent N-methyl-2-pyrrolidinone.

10. The formulation according to claim 1 which contains an ester of polyethylene glycol selected from the group consisting of

$$CH_3\text{-}(CH_2)_a\text{-}CO\text{-}(OCH_2CH_2)_n\text{-}O\text{-}R_6 \qquad \text{(II)}$$

wherein i) $R_6$ is hydrogen, a is 8 to 16 and n is 4 to 20, or ii) $R_6$ is $-CO-(CH_2)_b-CH_3$, b is 8 to 16, a is 8 to 16 and n is 4 to 150,

$$CH_3\text{-}(CH_2)_c\text{-}CH{=}CH\text{-}(CH_2)_d\text{-}CO\text{-}(OCH_2CH_2)_m\text{-}OH \qquad \text{(III)}$$

wherein m is 4 to 20, c is 6 to 8 and d is 6 to 8, and

$$HO\text{-}(CH_2CH_2O)_e\text{-}CO\text{-}(CH_2)_k\text{-}CO\text{-}(OCH_2CH_2)_f\text{-}OH \qquad \text{(IV)}$$

wherein k is 3 to 6, e is 4 to 20 and f is 4 to 20.

11. The formulation according to claim 1 which contains the polyethylene glycol ester PEG 200 dilaurate.

12. The formulation according to claim 1 which contains a non-ionic surfactant selected from the group consisting of

$$CH_3\text{-}(CH_2)_g\text{-}CO\text{-}(OCH_2CH_2)_p\text{-}OH \qquad \text{(V)}$$

wherein g is 8 to 16 and p is 1 or 2,

$$CH_3\text{-}(CH_2)_q\text{-}CH{=}CH\text{-}(CH_2)_r\text{-}CO\text{-}(OCH_2CH_2)_s\text{-}OH \qquad (VI)$$

wherein q is 6 to 8; r is 6 to 8, and s is 1 or 2, and

$$CH_3\text{-}(CH_2)_h\text{-}CO\text{-}OCH_2CH_2O\text{-}CO\text{-}(CH_2)_i\text{-}CH_3 \qquad (VII)$$

wherein h and i each are 8 to 16.

**13.** The formulation according to claim 1 which contains a non-ionic surfactant selected from the group consisting of ethoxylated triglycerides.

**14.** The formulation according to claim 1 which contains as non-ionic surfactant ethoxylated castor oil.

**15.** The formulation according to claim 1 which contains 0.1 to 25 % by weight of at least one bio-active substituted benzoylphenyl urea which is sparingly soluble in water, 5 to 25 % by weight of at least one N-substituted pyrrolidinone solvent, 30 to 60 % by weight of at least one ester of polyethylene glycol and 20 to 60 % by weight of at least one non-ionic surfactant selected from an ethoxylated ester of a fatty acid or a triglyceride, and optionally, 0.1 to 0.3 % by weight of ethyl cellulose.

**16.** The formulation according to claim 1 which contains 0.1 to 25 % by weight of a bio-active substituted benzoylphenyl urea which is sparingly soluble in water selected from the group consisting of N-(4-chlorophenyl-N'-(2,6-difluorobenzoyl)urea, N-(2,5-dichloro-4-(1,1,2,3,3,3-hexafluoropropoxy)phenyl)-N'-(2,6-difluorobenzoyl)urea and N-(4-chloro-3-(3-chloro-5-trifluoromethyl-pyridyl-2-oxy)phenyl)-N'-(2,6-difluorobenzoyl) urea, 5 to 25 % by weight of N-methyl-2-pyrrolidinone, 30 to 60 % by weight of a fatty acid ester of polyethylene glycol containing 4 to 150 EO, 20 to 60 % by weight of ethoxylated castor oil and 0 to 0.3 % by weight of ethyl cellulose.

**17.** A method of solubilizing in water a bio-active substituted benzoyl phenyl urea which is sparingly soluble in water or a mixture of such ureas which method comprises combining said urea or said mixture of ureas with

(ii) at least one N-substituted pyrrolidinone solvent;

(iii) at least one ester of polyethylene glycol; and

(iv) at least one non-ionic surfactant selected from an ethoxylated ester of a fatty acid or a triglyceride.

**18.** A method for enhancing absorption and maintaining adequate blood levels in warm-blooded animals of bio-active substituted benzoylphenyl ureas which are sparingly soluble in water, which method comprises administering to said warm-blooded animals orally, directly into the gastro-intestinal tract or parenterally a formulation according to claim 1.

**19.** A method for combatting ectoparasites which attack warm-blooded animals, which method comprises administering to the animal to be treated a formulation according to claim 1.

**20.** The method according to claim 19, which method comprises administering to the animal to be treated an amount of 1 to 20 mg per kg body weight of a water insoluble bio-active substituted benzoylphenyl urea as consitituent of a formulation described in claim 1.